# EUROPEAN PATENT APPLICATION

(11) **EP 4 252 860 A1**
(43) Date of publication of application: **04.10.2023**
(21) Application number: 22305419.8
(22) Date of filing: 31.03.2022
(51) Int. Cl.: A61Q 1/06, A61K 8/92, A61K 8/37, A61K 8/34

(54) **ANHYDROUS FORMULATIONS SUITABLE AS LIP BALMS**

(71) Applicant: Johnson & Johnson Consumer Inc., Skillman, NJ 08558 (US)
(72) Inventor: LATOMBE, Alice, 27100 Val-de-Reuil (FR); FREVILLE, Vianney, 27100 Val-de-Reuil (FR)
(74) Representative: Carpmaels & Ransford LLP

(57) **Abstract**

Described are solid anhydrous formulations suitable for lip care comprising: greater than 40% to less than 60% of an oil mixture comprising at least 70% of C8-10 fatty acids; greater than 15% to less than 25% of a wax mixture comprising at least 20% of esters of C20-24 fatty acids with glycerol; and greater than 20% to less than 40% of at least one butter, wherein the at least one butter comprises a natural butter which is solid at 20°C and has a melting point below 50°C., wherein all % are expressed in weight %.

## Description

### FIELD

The invention generally relates to skin care and more particularly to lip care. In particular, the invention relates to a solid anhydrous formulation for lip care comprising an oil mixture, a wax mixture and a butter mixture. The invention also relates to a method of treating the lips using said solid anhydrous formulation.

### BACKGROUND

Lip balm for lip care and hydration are well known products and are used throughout the year by many consumers.

In the recent years, consumers demands have evolved toward more natural products. These changes in demands may be driven, for example, by ecological concerns regarding the sourcing of raw material, or by health concerns regarding potential side effects of ingredients such as preservatives.

Manufacturers are constantly trying to improve the formulation of their products to match consumer expectations. Consequently, it is highly desirable to develop lip balm formulations without synthetic ingredients (such as ethylhexyl stearate), mineral oil and preservatives (such as butylated hydroxytoluene).

However, these more natural products often have inferior sensory characteristics and can be unpleasant to apply. They are often not as stable and not be robust enough for the manufacturing process. There is thus an ongoing need for natural personal care products, especially natural lip balms.

### SUMMARY OF THE INVENTION

One aspect of the invention pertains to a solid anhydrous formulation for lip care comprising:
- greater than 40% to less than 60% of an oil mixture comprising at least 70% of C8-10 fatty acids;
- greater than 15% to less than 25% of a wax mixture comprising at least 20% of esters of C20-24 fatty acids with glycerol; and
- greater than 20% to less than 40% of at least one butter, wherein the at least one butter comprises a natural butter which is solid at 20°C and has a melting point below 50°C., wherein all % are expressed in weight %.

In one or more embodiments, the c8-10 fatty acids comprise caprylic/capric triglycerides.

in some embodiments, wherein esters of c20-24 fatty acids with glycerol comprise glyceryl behenate. In one or more embodiments, the natural butter which is solid at 20°C and a melting point below 50°C comprises shea butter. In some embodiments, wherein the at least one butter comprises at least two butters and the natural butter which is solid at 20°C and has a melting point below 50°C comprises at least 50% of the total butter content. In one or more embodiments, the oil mixture further comprises an oil component selected from the group consisting of: triethylhexanoin, triheptanoin, hexyldecyl laurate, hexyldecanol, isodecyl oleate, cocoglycerides, cetearyl isononanoate, ethylhexyl stearate, octyldodecyl myristate, isopropyl isostearate, and mixtures thereof. In some embodiments, the oil mixture further comprises from 0.05% to 5% of an oil selected from the group consisting of: prunus amygdalus dulcis (sweet almond) oil, or persea gratissima (avocado) oil, or rosa damascena flower extract, or a combination thereof; all % are expressed in weight %. In one or more embodiments, the oil mixture comprises more than 80%, preferably more than 90%, preferably more than 95% of caprylic/capric triglycerides, and wherein all % are expressed in weight %. In some embodiments, the wax mixture further comprises at least 40% of synthetic beeswax, and wherein all % are expressed in weight %. In one or more embodiments, the wax mixture further comprises from 1% to 10%, preferably from 1% to 5%, of Copernicia Cerifera (Carnauba) Wax, and wherein all % are expressed in weight %. In some embodiments, the wax mixture further comprises wax selected from the group consisting of synthetic beeswax, tribehenin, copernicia cerifera wax, glyceryl behenate, cetearyl alcohol, hydrogenated rapeseed oil, cera microcristallina, synthetic wax, and combinations thereof.

In one or more embodiments, the butter mixture further comprises less than 50% of a combination of olus oil and hydrogenated vegetable oil and candelilla cera, and wherein all % are expressed in weight %. In some embodiments, the butter mixture further comprises butter selected among: vegetable petrolatum; glyceryl oleate; olus oil; hydrogenated vegetable oil; candelilla cera; or combination thereof. In one or more embodiments, the solid anhydrous formulation for lip care of any of the preceding claims, further comprising less than 5%, preferably less than 1% of glycerin; all % are expressed in weight %. In some embodiments, the formulation contains ingredients which are not chemically modified from a natural origin source. In one or more embodiments, wherein the formulation comprises:
- greater than 40% to less than 60% of an oil mixture comprising at least at least 90% of Caprylic/Capric triglycerides;
- greater than 15% to less than 25% of a wax mixture comprising at least 20% of Glyceryl Behenate; and at least 40% of synthetic beeswax;
- from 25% to 35% of at least one butter, wherein the at least one butter comprises at least 50% of shea butter; and from 10% to less than 50% of a combination of olus oil and hydrogenated vegetable oil and candelilla cera,
wherein all % are expressed in weight %. In some embodiments, the formulation has a static hardness comprised between 70 gf to 200 gf, preferably 80gf to 150gf. In one or more embodiments, the formulation has a drop point comprised between 55°C to 80°C.

Another aspect of the invention pertains to a use of a solid anhydrous formulation according to any of the above embodiments, in any combination for the preparation of a lip stick balm.

Another aspect of the invention pertains to a use of a solid anhydrous formulation according to any of the above embodiments, in any combination, for the make up or cosmetic care, for example hydration, of the lips.

Another aspect of the invention pertains to a method of treatment of the lips, the method comprising applying to the lips a formulation according to any of the above embodiments, in any combination.

### DETAILED DESCRIPTION OF EMBODIMENTS OF THE INVENTION

As used herein, "cosmetically / dermatologically acceptable" means suitable for use in contact with tissues (e.g., the skin or hair) without undue toxicity, incompatibility, instability, irritation, and/or allergic response, and the like.

As used herein, the term "safe and effective amount" means an amount sufficient to induce the desired effect, but low enough to avoid serious side effects. The safe and effective amount of the compound, extract, or composition will vary with, e.g., the age, health and environmental exposure of the end user, the duration and nature of the treatment, the specific extract, ingredient, or composition employed, the particular pharmaceutically-acceptable carrier utilized, and like factors.

As used herein, "essentially free" or "substantially free" of an ingredient means containing less than 0.1 weight percent, or less than 0.01 weight percent, or none of an ingredient.

One aspect of the invention pertains to a solid anhydrous formulation for lip care that comprises:
- greater than 40% to less than 60% of an oil mixture comprising at least 70% of C8-10 fatty acids triglycerides;
- greater than 15% to less than 25% of a wax mixture comprising at least 20% of esters of C20-24 fatty acids with glycerol; and
- greater than 20% to less than 40% of at least one butter, wherein the at least one butter comprises at a natural butter which is solid at 20°C and has a melting point below 50°C, wherein all % are expressed in weight %. By "anhydrous" it is meant that the solid formulation for lip care contains less than 1% of water; preferably less than 0.5% of water; even more preferably less than 0.1% of water, all % expressed in weight %. In some embodiments, the solid formulation is substantially free of water, or contains no water.

It has been surprisingly discovered that the above combination of ingredients in the above concentration ranges provides a superior lip care formulation. The lip care formulation contains natural ingredients, and yet still maintains sufficient hardness to survive the manufacturing process (demonstrated through drop point) and has good application/sensory properties (demonstrated through static hardness).

Static hardness values give an indication about the consumer sensory feeling when the solid anhydrous formulation is applied to skin (e.g., lips). If the static hardness is too high, then the formulation is too rigid. Static hardness is defined by the force required to cut the stick with a wire, the wire is connected to a dynamometer to measure the force applied.

A formulation too rigid may not glide upon application or may not melt enough to be pleasant to the consumer. Generally, consumers prefer a static hardness at or below about 200 gf, and some consumers may also prefer a static hardness at 150 gf or below. On the other hand, if the static hardness is too low the formulation may not be able to hold its shape, for example a stick. The formulation may also melt too quickly and may provide an unpleasant sensory experience to the consumer. Generally, consumers prefer a static hardness of at least about 70 gf. The static hardness for the solid anhydrous formulation suitable for lip care according to one or more embodiments of the invention may range from between about 70 gf to about 200 gf, specifically about 80 gf to about 150 gf or about 120 gf.

Drop point values give an indication about the solid anhydrous formulation manufacturing process. The dropping point is defined as the temperature at which the first drop of a melted sample of the substance under investigation flows through the 2.8 mm opening of a standard dropping point sample cup on slow heating.

If the drop point is too high it may be difficult to melt the formulation and mold it into the desired shape, for example a stick shape. Also, if the drop point is too low, the formulation may be unstable overtime and may melt too easily if close to a heat source or if the external environment gets too hot like in the summer in certain areas. The drop point value for the solid anhydrous formulation suitable for lip care according to one or more embodiments of the invention may range from between about 55°C to about 80°C, or more preferably 66°C.

In one or more embodiments, the solid anhydrous formulation for lip care according to the present invention has a static hardness comprised between 70 gf to 200 gf, preferably 80 gf to 150 gf, and a drop point comprised between 55°C to 80°C, or 55°C to 66°C.

### Oil

The compositions described herein comprise greater than 40% to less than 60% of an oil mixture comprising at least 70% of C8-10 fatty acids triglycerides. By "Oil" it is meant a liquid at 20°C that is insoluble in water. The oil is thought to provide softness and protect the skin.

C8-10 fatty acid triglycerides are triglycerides derived from a mixture of C8-10 fatty acids (i.e., those with a principal carbon atom chain comprising from 8 to 10 consecutive carbon atoms). For example, the C8-10 fatty acids triglycerides may comprise Caprylic/Capric triglycerides. Suitable fatty acid triglycerides are available as Myritol^{®} 312 from BASF.

The oil mixture of the solid anhydrous formulation for lip care according to one or more embodiments of the invention may further comprise an oil component selected from the group consisting of: triethylhexanoin, triheptanoin, hexyldecyl laurate, hexyldecanol, isodecyl oleate, cocoglycerides, cetearyl isononanoate, ethylhexyl stearate, octyldodecyl myristate, isopropyl isostearate, and mixtures thereof.

The oil mixture of the solid anhydrous formulation for lip care according to the invention may comprise an oil of natural origin selected from the group consisting of: Prunus Amygdalus Dulcis (Sweet Almond) Oil, or Persea Gratissima (Avocado) Oil, or Rosa Damascena Flower Extract, or a combination thereof; wherein all % are expressed in weight %.

The solid formulation should contain at least about 40%, preferably more than about 40% of the oil, and no more than about 60%, preferably less than about 60% of the oil mixture.

Preferably, the oil mixture of the solid anhydrous formulation for lip care according to the invention may comprise more than 80%, preferably more than 90%, preferably more than 95% of Caprylic/Capric triglycerides; wherein all % are expressed in weight %. In embodiments containing natural oils, the oil mixture of the solid anhydrous formulation for lip care according to the invention may comprise said natural oil in an amount of about 0.01 to about 1 %; wherein all % are expressed in weight %.

### Wax

The solid anhydrous formulations described herein comprise from about 15% to about 25% of a wax mixture. By "Wax" it is meant a semisolid substance at room temperature (20°C) that melts to become a low viscosity liquid at around 50 °C, and is insoluble in water. It is thought the wax has the function to increase viscosity and bring consistency to improve final texture and adhesion of the product.

The wax mixture comprises at least about 20% of esters of C20-24 fatty acids with glycerol. Such C20-24 fatty acids are those which have a principal carbon atom chain comprising from 20 to 24 consecutive carbon atoms. A mixture of these fatty acids may be esterified with glycerol to provide the esters of C20-24 fatty acids with glycerol. For example, the esters of C20-24 fatty acids with glycerol may comprise Glyceryl Behenate, available as Compritol 888 CG pellets from Gattefosse.

The solid formulation in accordance with one or more embodiments of the invention should contain at least 15%, preferably more than 15% of the wax mixture, and no more than 25%, preferably less than 25% of the wax mixture.

Preferably, the said wax mixture may comprise additional waxes selected from the group consisting of synthetic beeswax, tribehenin, copernicia cerifera (carnauba) wax, glyceryl behenate, cetearyl alcohol, hydrogenated rapeseed oil, cera microcristallina (or microcristalline wax), synthetic wax, and combinations thereof.

The wax mixture of the solid anhydrous formulation for lip care according to the invention may further comprise from 1% to 10%, preferably from 1% to 5%, of additional waxes, wherein all % are expressed in weight %.

For example, in one or more embodiments, the wax mixture of the solid anhydrous formulation for lip care according to the invention may further comprise from 1% to 10%, preferably from 1% to 5%, of copernicia cerifera (carnauba) wax, and wherein all % are expressed in weight %.

### Butter

The solid anhydrous formulations comprise 20% to 40% of at least one butter. By "Butter" it is meant a natural butter which is solid at 20°C and has a melting point below 50°C. The butter is thought to bring melting properties to the product and help the application on the lips. While not wishing to be bound to any particular theory, it is thought that the at least one butter brings firmness to the solid formulation. In some embodiments, the solid anhydrous formulation comprises at least 20%, preferably more than 20%, of butter in the solid formulation, which helps to makes the formulation rigid enough to be molded.

Preferably, the said butter mixture may comprise butter selected among: shea butter, vegetable petrolatum; glyceryl oleate; olus oil; hydrogenated vegetable oil; candelilla cera; or combination thereof that is solid at 20°C and has a melting point below 50°C. preferably, the said natural butter comprises shea butter. when present, the shea butter may comprise at least 50% of the total butter content.

In one or more embodiments, the butter mixture of the solid anhydrous formulation for lip care according to the invention may further comprise less than 50% of a combination of Olus Oil and Hydrogenated Vegetable Oil and Candelilla Cera, and wherein all % are expressed in weight %.

The wax mixture of the solid anhydrous formulation for lip care according to the invention may further comprise at least 40% of synthetic beeswax, and wherein all % are expressed in weight %.

In a further aspect the solid anhydrous formulation for lip care according to the invention contains ingredients which are not chemically modified from a natural origin source. In other words, it is preferred from a consumer perspective to have a formulation that does not contain ingredient which have been modified by chemical processes to change their naturally occurring chemical structure. In one or more embodiments, the ingredients may be naturally sourced or chemically synthetized to have a structure identical to the natural ingredient. For example, the synthetic beeswax may not be naturally sourced but is however chemically equivalent to the natural product and is therefore acceptable in the context of the present invention.

The above variations may be combined any suitable way. For example, in a preferred embodiment, the solid anhydrous formulation for lip care according to the present invention may have a formulation comprising:
from 45% to 55% of an oil mixture comprising at least 90% of Caprylic/Capric triglycerides;
greater than 15% to less than 25% of a wax mixture comprising at least one wax, wherein the wax mixture comprises at least 20% of Glyceryl Behenate; and at least 40% of synthetic beeswax;
from 25% to 35% of a butter mixture comprising at least one butter, wherein the butter mixture comprises at least 50% of shea butter; and from 10% to less than 50% of a combination of Olus Oil and Hydrogenated Vegetable Oil and Candelilla Cera,
wherein all % are expressed in weight %.

Preferably the solid anhydrous formulation for lip care comprises from 17,5% to 22,5% of a wax mixture comprising at least one wax, wherein the wax mixture comprises at least 20% of Glyceryl Behenate; and at least 40% of synthetic beeswax.

### Other Ingredients

The compositions of the present invention may further comprise any of a variety of additional cosmetically active agents. Examples of suitable additional active agents include: skin lightening agents, darkening agents, additional anti-aging agents, tropoelastin promoters, collagen promoters, anti-acne agents, shine control agents, anti-microbial agents such as anti-yeast agents, anti-fungal, and anti-bacterial agents, anti-inflammatory agents, anti-parasite agents, external analgesics, sunscreens, photoprotectors, antioxidants, keratolytic agents, detergents/surfactants, moisturizers, nutrients, vitamins, energy enhancers, anti-perspiration agents, astringents, deodorants, hair removers, hair growth enhancing agents, hair growth delaying agents, firming agents, hydration boosters, efficacy boosters, anti-callous agents, agents for skin conditioning, anti-cellulite agents, odor-control agents such as odor masking or pH changing agents, and the like.

Examples of various suitable additional cosmetically acceptable actives include hydroxy acids; benzoyl peroxide; D-panthenol; UV filters such as but not limited to avobenzone (Parsol 1789), bisdisulizole disodium (Neo Heliopan AP), diethylamino hydroxybenzoyl hexyl benzoate (Uvinul A Plus), ecamsule (Mexoryl SX), methyl anthranilate, 4-aminobenzoic acid (PABA), cinoxate, ethylhexyl triazone (Uvinul T 150), homosalate, 4-methylbenzylidene camphor (Parsol 5000), octyl methoxycinnamate (Octinoxate), octyl salicylate (Octisalate), padimate O (Escalol 507), phenylbenzimidazole sulfonic acid (Ensulizole), polysilicone-15 (Parsol SLX), trolamine salicylate, Bemotrizinol (Tinosorb S), benzophenones 1-12, dioxybenzone, drometrizole trisiloxane (Mexoryl XL), iscotrizinol (Uvasorb HEB), octocrylene, oxybenzone (Eusolex 4360), sulisobenzone, bisoctrizole (Tinosorb M), titanium dioxide, zinc oxide; carotenoids; free radical scavengers; spin traps; retinoids and retinoid precursors such as retinol, retinoic acid and retinyl palmitate; ceramides; polyunsaturated fatty acids; essential fatty acids; enzymes; enzyme inhibitors; minerals; hormones such as estrogens; steroids such as hydrocortisone; 2-dimethylaminoethanol; copper salts such as copper chloride; peptides containing copper such as Cu:Gly-His-Lys, coenzyme Q10; amino acids such a proline; vitamins; lactobionic acid; acetyl-coenzyme A; niacin; riboflavin; thiamin; ribose; electron transporters such as NADH and FADH2; and other botanical extracts such as oat, aloe vera, Feverfew, Soy, Shiitake mushroom extracts, and derivatives and mixtures thereof.

If present, any additional cosmetically active agent may be present in a composition in any suitable amount, for example, in an amount of from about 0.0001% to about 20% by weight of the composition, e.g., about 0.001% to about 10% such as about 0.01% to about 5%. In some embodiments, in an amount of 0.1% to 5% and in other embodiments from 1% to 2%.

Compositions of the present invention may include a cosmetically effective amount of one or more additional anti-inflammatory compounds. Examples of suitable anti-inflammatory agents include substituted resorcinols, (E)-3-(4-methylphenylsulfonyl)-2-propenenitrile (such as "Bay 11-7082," commercially available from Sigma-Aldrich of St. Louis, Missouri), tetrahydrocurcuminoids (such as Tetrahydrocurcuminoid CG, available from Sabinsa Corporation of Piscataway, NJ), extracts and materials derived from the following: *Phellodendron amurense* Cortex Extract (PCE), Non-Denatured Soy (*Glycine max*), Feverfew (*Tanacetum parthenium*), Ginger (*Zingiber officinale*), Ginkgo (*Ginkgo biloba*), Madecassoside (*Centella asiatica* extract ingredient), Cotinus *(Cotinus coggygria),* Butterbur Extract (*Petasites hybridus*)*,* Goji Berry (*Lycium barbarum*)*,* Milk Thistle Extract (*Silybum marianum*), Honeysuckle (*Lonicera japonica*), Basalm of Peru (*Myroxylon pereirae*), Sage (*Salvia officinalis*), Cranberry Extract (*Vaccinium oxycoccos*), Amaranth Oil (*Amaranthus cruentus*), Pomegranate (*Punica granatum*), Yerbe Mate (*Ilex paraguariensis* Leaf Extract), White Lily Flower Extract (*Lilium candidum*), Olive Leaf Extract (*Olea europaea*), Phloretin (apple extract), Oat Flour (*Aveena sativa*), Lifenol (Hops: *Humulus lupulus*) Extract, Bugrane P (*Ononis spinosa*), Licochalcone (Licorice: *Glycyrrhiza inflate* extract ingredient), Symrelief (Bisabolol and Ginger extract), combinations of two or more thereof, and the like.

In one embodiment, the anti-inflammatory agent is a resorcinol. Particularly suitable substituted resorcinols include 4-hexyl resorcinol and 4-octylresorcinol, particularly 4-hexyl resorcinol. 4-Hexyl resorcinol is commercially available as "SYNOVEA HR" from Sytheon of Lincoln Park, NJ. 4-Octylresorcinol is commercially available from City Chemical LLC of West Haven, Connecticut.

By "extracts of feverfew," it is meant extracts of the plant *"Tanacetum parthenium,"* such as may be produced according to the details set for the in US Patent No. 7,537,791, entitled "PARTHENOLIDE FREE BIOACTIVE INGREDIENTS FROM FEVERFEW (TANACETUM PARTHENIUM) AND PROCESSES FOR THEIR PRODUCTION." One particularly suitable feverfew extract is commercially available as about 20% active feverfew, from Integrated Botanical Technologies of Ossining, NY.

A variety of other materials may also be present in the compositions of the present invention.

In one or more embodiments, the composition comprises one or more topical ingredients selected from the group consisting of: surfactants, chelating agents, emollients, humectants, conditioners, preservatives, opacifiers, fragrances and the like.

What is meant by an emollient is a compound that helps to maintain the soft, smooth, and pliable appearance of the skin (*e*.*g*., by remaining on the skin surface or in the stratum corneum to act as a lubricant). Examples of suitable emollients include those found in Chapter 35, pages 399-415 (Skin Feel Agents, by G Zocchi) in Handbook of Cosmetic Science and Technology (edited by A. Barel, M. Paye and H. Maibach, Published in 2001 by Marcel Dekker, Inc New York, NY), and include, but are not limited to, petrolatum, hexyldecyl stearate and plant, nut, and vegetable oils such as macadamia nut oil, sunflower oil, almond oil, rice bran oil, grape seed oil, palm oil, prim rose oil, hydrogenates peanut oil, and avocado oil.

What is meant by a humectant is a compound intended to increase the water content of the top layers of skin (*e*.*g*., hygroscopic compounds). Examples of suitable humectants include those found in Chapter 35, pages 399-415 (Skin Feel Agents, by G Zocchi) in Handbook of Cosmetic Science and Technology (edited by A. Barel, M. Paye and H. Maibach, Published in 2001 by Marcel Dekker, Inc New York, NY) and include, but are not limited to, glycerin, sorbitol or trehalose (e.g., α,α- trehalose, β,β-trehalose, α,β-trehalose) or a salt or ester thereof (*e*.*g*., trehalose 6-phosphate). In one or more embodiments, the solid anhydrous formulation for lip care may further comprise less than 5%, preferably less than 1% of glycerin; all % are expressed in weight %. Amounts above 5%, are thought to destabilize the composition.

What is meant by a surfactant is a surface-active agent intended to cleanse or emulsify. Examples of suitable surfactants include those found in Chapter 37, pages 431-450 (Classification of surfactants, by L. Oldenhove de Guertechin) in Handbook of Cosmetic Science and Technology (edited by A. Barel, M. Paye and H. Maibach, Published in 2001 by Marcel Dekker, Inc., New York, NY) and include, but are not limited to anionic surfactants such as sulfates, cationic surfactants such as betaines, amphoteric surfactants such as sodium coco glycinate, nonionic surfactants such as alkyl polyglucosides.

Examples of suitable chelating agents include those which are capable of protecting and preserving the compositions of this invention. In one or more embodiments, the chelating agent is ethylenediaminetetraacetic acid ("EDTA"), and more specifically is tetrasodium EDTA, available commercially from Dow Chemical Company of Midland, Michigan under the trade name, "Versene 100XL."

Suitable preservatives include, for example, parabens, quaternary ammonium species, phenoxyethanol, benzoates, DMDM hydantoin, organic acids and are present in the composition in an amount, based upon the total weight of the composition, from about 0 to about 1 percent or from about 0.05 percent to about 0.5 percent.

Any of a variety of conditioners which impart additional attributes, such as gloss to the hair, are suitable for use in this invention. Examples include, but are not limited to, volatile silicone conditioning agent having an atmospheric pressure boiling point less than about 220°C. Examples of suitable volatile silicones nonexclusively include polydimethylsiloxane, polydimethylcyclosiloxane, hexamethyldisiloxane, cyclomethicone fluids such as polydimethylcyclosiloxane available commercially from Dow Corning Corporation of Midland, Michigan under the tradename, "DC-345" and mixtures thereof, and specifically include cyclomethicone fluids. Other suitable conditioners include cationic polymers, including polyquarterniums, cationic guar, and the like.

Any of a variety of commercially available pearlescent or opacifying agents are suitable for use in the composition. Examples of suitable pearlescent or opacifying agents include, but are not limited to, mono or diesters of (a) fatty acids having from about 16 to about 22 carbon atoms and (b) either ethylene or propylene glycol; mono or diesters of (a) fatty acids having from about 16 to about 22 carbon atoms (b) a polyalkylene glycol of the formula: HO-(JO)a-H, wherein J is an alkylene group having from about 2 to about 3 carbon atoms; and a is 2 or 3; fatty alcohols containing from about 16 to about 22 carbon atoms; fatty esters of the formula: KCOOCH2L, wherein K and L independently contain from about 15 to about 21 carbon atoms; inorganic solids insoluble in the shampoo composition, and mixtures thereof.

Any fragrance compositions suitable for use on skin may be used in the composition according to the present invention.

Preferably the solid anhydrous formulation does not contain mattifying agents. As used herein, "mattifying agents" refers to ingredients which reduce the shine and/or glossiness of a composition. Examples of mattifying agents includes be particulate materials. By "particulate materials" it is meant moieties that do not dissolve in the liquid vehicle, but rather and form discrete units greater than about 0.2 microns, but less than about 1000 microns, such as may be suspended within the skin care composition. The particulate material may be a hard inorganic particulate (without or without hydrophobic coatings or surface modification) including, for example, oxides such as oxides of silica (including fumed silica, precipitated silica, and colloidal silica), titanium dioxide, or other chemically produced or mined oxides, talc, mica, or aluminosilicates, and the like. The particulate material may be a fine particulate. By "fine particulate" it is meant a particulate that is generally capable of forming fine, discrete domains (e.g. less than about 200 microns, such as from about 0.2 microns to about 100 microns, such as from about 1 micron to about 50 microns, such as from about 1 micron to about 20 microns in the film). Other particulates of note include inorganic particulates such as silica gels, aluminum silicates, and fumed silicas such as surface modified or silylated fumed silicas such as Aerosil R812S available from Degussa AG (Piscataway, N.J.).

### Uses, Methods and Forms

The compositions of the present invention can also be formulated into a solid formulation (*e*.*g*., a stick, soap bar composition, powder, or wipe). The composition of the present invention can also be combined with a solid, semi-solid, or dissolvable substrate (*e*.*g*., a wipe, mask, pad, glove, or strip).

The composition may be applied topically. Such topical application may be to any skin in need of treatment on the body, for example skin of the face, lips, neck, chest, back, buttocks, arms, axilla, and/or legs. Any of the solid anhydrous formulations described herein may be suitable for the use of skin dryness, in particular dryness of the lips. In one or more embodiments, the present invention includes the solid anhydrous formulation for lip care as described above for the treatment of skin dryness, particularly for lips treatment. In a further embodiment, the solid anhydrous formulation according to the invention may be used for the preparation of a lip stick balm. In yet a further embodiment the solid anhydrous formulation according to the invention may be used for the make up or cosmetic care, for example hydration, of the lips.

In one or more embodiments, the present invention includes a method of treatment of the lips wherein the method comprises a step of applying to the lips a solid anhydrous formulation as disclosed above.

Any suitable method of applying the composition to the skin in need may be used. For example, the composition may be applied directly from a package to the skin in need, by hand to the skin in need, or may be transferred from a substrate such as a wipe or mask, or a combination of two or more thereof. In other embodiments, the composition may be applied via a dropper, tube, roller, spray, and patch or added to a bath or otherwise to water to be applied to the skin, and the like. The composition may be applied in a variety of manners or forms, including, without limitation, as a leave-on cream, mask, and /or serum.

While the foregoing description represent exemplary embodiments of the present invention, it will be understood that various additions, modifications and substitutions may be made therein without departing from the spirit and scope of the present invention. In particular, it will be clear to those skilled in the art that the present invention may be embodied in other specific forms, structures, arrangements, proportions, and with other elements, materials, and components, without departing from the spirit or essential characteristics thereof. One skilled in the art will appreciate that the invention may be used with many modifications of structure, arrangement, proportions, materials, and components and otherwise, used in the practice of the invention, which are particularly adapted to specific environments and operative requirements without departing from the principles of the present invention. The presently disclosed embodiments are therefore to be considered in all respects as illustrative and not restrictive, the scope of the invention being indicated by the appended claims, and not limited to the foregoing description. It will be appreciated that in the claims, the term "comprises/comprising" does not exclude the presence of other elements or steps. In addition, singular references do not exclude a plurality. The terms "a", "an", "first", "second", etc., do not preclude a plurality.

All percentages, parts and ratios are based upon the total weight of the composition of the present invention, unless otherwise specified. All such weights as they pertain to the listed ingredients are based on the level of the particular ingredient described and, therefore, do not include carriers or by-products that may be included in commercially available materials, unless otherwise specified.

As used herein, "essentially free" or "substantially free" of an ingredient means containing less than 0.1 weight percent, or less than 0.01 weight percent, or none of an ingredient.

To provide a more concise description, some of the quantitative expressions given herein are not qualified with the term "about". It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

To provide a more concise description, some of the quantitative expressions herein are recited as a range from about amount X to about amount Y. It is understood that wherein a range is recited, the range is not limited to the recited upper and lower bounds, but rather includes the full range from about amount X through about amount Y, or any amount or range therein.

### EXPERIMENTAL PART

### METHOD

Stick preparation: All ingredients were mixed, melted under stirring at 85°C, then molded into stick form. After 10 minutes sticks were removed from the mold. To reproduce the industrial conditions there is no cooling step before removal from the mold.

### Static hardness

The stick is stored at 25°C+/-2°C at least 24hours before the measurement.

A tungsten thread (0.25 mm diameter) is linked to a dynamometer (DFS II Series Digital Force Gauges, dynamometer from Ametek) and driven by a motor. The stick is placed horizontally and supported at its base by a ring. The thread cuts the stick in a vertical motion, parallel to the ring plane and 5mm away from the ring.

The dynamometer's captor measures the force in compression and result is stated in gf (or gram force). As a preparing step, the stick is stored at 25°C+/-2°C at least 24hours before the measurement.

### Drop point

Drop point is measured with a Dropping Point system DP90 with One Click technology from Melter Toledo.

The dropping point is defined as the temperature at which the first drop of a melted sample of the substance under investigation flows through the 2.8 mm opening of a standard dropping point sample cup on slow heating.

Dropping point measurements are possible only with samples which have a low enough viscosity at elevated temperature (molten or liquid state) to flow through the 2.8 mm diameter opening: fats, paraffin, waxes, oils, organic powders, lubricating greases and also non-crystalline substances, such as low molecular weight natural resins.

The dropping point event is detected via video recording and simultaneous image evaluation.

### MATERIALS

- Seatons Sunflower Oil REF-LQ-(JL), INCI: Helianthus annuus seed oil from Croda;
- Myritol 312 / Miglyol 812 N(F) , INCI: Caprylic/capric triglycerides from BASF or Cremer Oleo;
- Almond Oil BP 73, INCI: Amygdalus Dulcis (Sweet Almond) Oil from Olvea;
- REFINED & WINTERISED AVOCADO OI, INCI: Persea Gratissima (Avocado) Oil from Olvea;
- CremerGLYC Refined Glycerine 99,5% (Art,N deg 31099XX) , INCI: Glycerin from Cremer Oleo
- Rose de provence extrait huileux (preferred), INCI: Caprylic/Capric Triglyceride; Rosa Damascena Flower Extract from Greentech;
- Syncrowax BB-4, INCI: synthetic beeswax from Croda;
- Cerauba T1, INCI: Copernicia Cerifera (Carnauba) Wax from Baerlocher France;
- Compritol 888 CG pellets, INCI: Glyceryl Behenate from Gattefosse;
- Cetiol SB 45, INCI: Butyrospermum Parkii (Shea) Butter from BASF;
- Cegesoft VP, INCI: Olus Oil (and) Hydrogenated Vegetable Oil (and) Candelilla Cera from BASF;
- Crodabon CSA, INCI:Hydrogenated Castor Oil/Sebacic Acid Copolymer from Croda.

### RESULTS

1) In the first step the best ratio of an oil (caprylic/capric triglycerides) a wax (glyceryl behenate) and a butter (butyrospermum parkii (shea) butter) was investigated. Caprylic/capric triglycerides, glyceryl behenate and butyrospermum parkii (shea) butter were selected because they are natural ingredients, and they constitute a reasonable starting point to the development of a lip stick balm formulations.

Results of trials 1 to 7 are listed in table 1 below. Ratio of the oil, wax and butter are indicted in weight %.

**Table 1**

| | **Trial 1** | **Trial 2** | **Trial 3** | **Trial 4** | **Trial 5** | **Trial 6** | **Trial 7** |
|---|---|---|---|---|---|---|---|
| INCI | | | | | | | |
| *Caprylic*/*capric triglycerides* | 60 | 55 | 45 | 47,5 | 50 | 40 | 52,5 |
| *Glyceryl Behenate* | 15 | 25 | 15 | 25 | 20 | 25 | 15 |
| *Butyrospermum Parkii (Shea) Butter* | 25 | 20 | 40 | 27,5 | 30 | 35 | 32,5 |
| | | | | | | | |
| Comment on process | Too soft | Too soft | Too soft | Good | Good | Good | Too soft |
| Broken samples | 20 | 20 | 18 | 6 | 6 | 6 | 18 |
| Static hardness (gf) | 40,9 | 110,6 | 68,8 | 131,7 | 125,1 | 221,3 | 67,5 |
| Drop point (°C) | 60,9 | 64,7 | 61,6 | 64,2 | 63,8 | 64,5 | 62,3 |
| Application - after 24h | Too soft | Good | Good | Hard | Good | Average | Too soft |

Regarding the "Comment on process" : "Too soft" means that the samples made were not rigid enough and had shape retention issues after unmolding. "Good" means that no unmolding issue was observed. Surprisingly Trial 2 had process issues although the static hardness was at 110gf. That problem is probably related to the low amount of butter, since butter is known to bring firmness to the stick.

"Broken sample" designates the number of lipstick samples that were broken when removed from the mold. It gives an indication about the robustness of the formulation during its manufacturing process and the wastes that will be generated. A high rate of broken sample is inacceptable. Samples of Trials 1, 2, 3 and 7 were too fragile and broke too often when unmolded.

"Application - after 24h" relates to sensory observations about application of the samples made 24h after unmolding. "Too soft" means that the stick melt to quickly and don't hold its shape when applied on the lips. On the contrary "Hard" means that it didn't melt enough and don't glide when applied on the lips. "Good" means that the sensory feeling is acceptable with a stick gliding but not losing its shape, melting enough to impart a good sensory experience. "Average" means that the results are acceptable but inferior to "Good".

The best properties in terms of processability and sensory feeling were observed for formulations having a Static hardness between 70gf to 200gf, and a drop point between 55°C to 80°C, or more preferably 55°C to 66°C.

Trial 5 gave the best combination of results from a sensory perspective and processability (unmolding, drop point, and batch trial), therefore it was selected as starting point for the second step of the formulation design.

2) In the second step, based on the preferred ratio defined according to Trial 5, different combinations of oils, waxes and butter were investigated. Formulations tested are presented in tables 2 and 3.

Trial 5 is repeated in table 2 as a reference point.

Trial 8 is comparative because the oil mixture comprises only 50% of C8-10 fatty acids triglycerides (with the remainder as helianthus annus seed oil). Although the resulting lipstick had an acceptable hardness (70,8) it was too soft and many samples broke when removed from the mold.

For each ingredient, additionally to their supposed function, it is indicated if they are considered to belong to the oil, wax or butter category.

Miscellaneous category regroups the other ingredients such as antioxidant, fragrance, flavor and colorant which do not belong to oil, wax or butter.

**Table 2**

| | | Trial 5 | Trial 8 (Comp) | Trial 9 | Trial 10 | Trial 11 |
|---|---|---|---|---|---|---|
| | | | | | | |
| INCI | Function | % | % | % | % | % |
| *Helianthus annuus seed oil* | EMOLLIENT (OIL) | 0 | 25 | 0 | 0 | 0 |
| *Caprylic*/*capric triglycerides* | EMOLLIENT (OIL) | 50 | 25 | 50 | 50 | 50 |
| *Prunus Amygdalus Dulcis (Sweet Almond) Oil* | EMOLLIENT (OIL) | 0 | 0 | 0 | 0 | 0 |
| *Persea Gratissima (Avocado) Oil* | EMOLLIENT (OIL) | 0 | 0 | 0 | 0 | 0 |
| *Glycerin* | HUMECTANT (OIL) | 0 | 0 | 0 | 0 | 0 |
| *Caprylic*/*Capric Triglyceride; Rosa Damascena Flower Extract* | Skin Conditioner (OIL) | 0 | 0 | 0 | 0 | 0 |
| *synthetic beeswax* | VISCOSITY CONTROLLER (WAX) | 0 | 10 | 10 | 15 | 15 |
| *Copernicia Cerifera (Carnauba) Wax* | EMOLLIENT (WAX) | 0 | 1 | 1 | 1 | 1 |
| *Glyceryl Behenate* | VISCOSITY CONTROLLER (WAX) | 20 | 10 | 10 | 5 | 5 |
| *Butyrospermum Parkii (Shea) Butter* | BUTTER (BUTTER) | 30 | 15 | 15 | 15 | 15 |
| *Olus Oil (and) Hydrogenated Vegetable Oil (and) Candelilla Cera* | STRUCTURING AGENT (BUTTER) | 0 | 8 | 8 | 8 | 13 |
| *Hydrogenated Castor Oil*/*Sebacic Acid Copolymer* | STRUCTURING AGENT (BUTTER) | 0 | 5 | 5 | 5 | 0 |
| Miscellaneous | - | 0 | 1 | 1 | 1 | 1 |
| | | | | | | |
| Static hardness (gf) | | 125,1 | 70,8 | 71,3 | 109,2 | 108,3 |
| Drop point (°C) | | 63,8 | 59,2 | 59,3 | 59,6 | 58,4 |

**Table 3**

| | | Trial 12 | Trial 13 | Trial 14 | Trial 15 |
|---|---|---|---|---|---|
| | | | | | |

| INCI | Function | % | % | % | % |
|---|---|---|---|---|---|
| *Helianthus annuus seed oil* | EMOLLIENT (OIL) | 0 | 0 | 0 | 0 |
| *Caprylic*/*capric triglycerides* | EMOLLIENT (OIL) | 49,9 | 49,489 | 48,9 | 46,342 |
| *Prunus Amygdalus Dulcis (Sweet Almond) Oil* | EMOLLIENT (OIL) | 0 | 0,5 | 0,5 | 0 |
| *Persea Gratissima (Avocado) Oil* | EMOLLIENT (OIL) | 0,1 | 0 | 0,1 | 0 |
| *Glycerin* | HUMECTANT (OIL) | 0 | 0 | 0,5 | 0 |
| *Caprylic*/*Capric Triglyceride; Rosa Damascena Flower Extract* | Skin Conditioner (OIL) | 0 | 0 | 0 | 0,1 |
| *synthetic beeswax* | VISCOSITY CONTROLLER (WAX) | 15 | 15 | 15 | 15 |
| *Copernicia Cerifera (Carnauba) Wax* | EMOLLIENT (WAX) | 1 | 1 | 1 | 1 |
| *Glyceryl Behenate* | VISCOSITY CONTROLLER (WAX) | 5 | 5 | 5 | 5 |
| *Butyrospermum Parkii (Shea) Butter* | BUTTER (BUTTER) | 15 | 15 | 15 | 15 |
| *Olus Oil (and) Hydrogenated Vegetable Oil (and) Candelilla Cera* | STRUCTURING AGENT (BUTTER) | 13 | 13 | 13 | 13 |
| *Hydrogenated Castor Oil*/*Sebacic Acid Copolymer* | STRUCTURING AGENT (BUTTER) | 0 | 0 | 0 | 0 |
| Miscellaneous | - | 1 | 1,011 | 1 | 4,558 |
| | | | | | |
| Static hardness (gf) | | 107,6 | 112,9 | 109,8 | 149,8 |
| Drop point (°C) | | 58,6 | 56,7 | 58,2 | 58,4 |

As can be seen from the above, the inventive examples displayed the best properties.

## Claims

1. A solid anhydrous formulation for lip care comprising:
• greater than 40% to less than 60% of an oil mixture comprising at least 70% of C8-10 fatty acids;
• greater than 15% to less than 25% of a wax mixture comprising at least 20% of esters of C20-24 fatty acids with glycerol; and
• greater than 20% to less than 40% of at least one butter, wherein the at least one butter comprises a natural butter which is solid at 20°C and has a melting point below 50°C,
wherein all % are expressed in weight %.

2. The solid anhydrous formulation for lip care of claim 1, wherein the C8-10 fatty acids comprise Caprylic/Capric triglycerides.

3. The solid anhydrous formulation for lip care of claim 1 or 2, wherein esters of C20-24 fatty acids with glycerol comprise Glyceryl Behenate.

4. The solid anhydrous formulation for lip care of any of the preceding claims, wherein the natural butter which is solid at 20°C and a melting point below 50°C comprises shea butter.

5. The solid anhydrous formulation for lip care of any of the preceding claims, wherein the at least one butter comprises at least two butters and the natural butter which is solid at 20°C and has a melting point below 50°C comprises at least 50% of the total butter content.

6. The solid anhydrous formulation for lip care of any of the preceding claims, wherein the oil mixture further comprises an oil component selected from the group consisting of: triethylhexanoin, triheptanoin, hexyldecyl laurate, hexyldecanol, isodecyl oleate, cocoglycerides, cetearyl isononanoate, ethylhexyl stearate, octyldodecyl myristate, isopropyl isostearate, and mixtures thereof.

7. The solid anhydrous formulation for lip care of any of the preceding claims, wherein the oil mixture further comprises from 0.05% to 5% of an oil selected from the group consisting of: Prunus Amygdalus Dulcis (Sweet Almond) Oil, or Persea Gratissima (Avocado) Oil, or Rosa Damascena Flower Extract, or a combination thereof; all % are expressed in weight %.

8. The solid anhydrous formulation for lip care of any of the preceding claims, wherein the oil mixture comprises more than 80%, preferably more than 90%, preferably more than 95% of caprylic/capric triglycerides, and wherein all % are expressed in weight %.

9. The solid anhydrous formulation for lip care of any of the preceding claims, wherein the wax mixture further comprises at least 40% of synthetic beeswax, and wherein all % are expressed in weight %.

10. The solid anhydrous formulation for lip care of any of the preceding claims, wherein the wax mixture further comprises from 1% to 10%, preferably from 1% to 5%, of copernicia cerifera wax, and wherein all % are expressed in weight %.

11. The solid anhydrous formulation for lip care of any of the preceding claims, wherein the wax mixture further comprises wax selected from the group consisting of synthetic beeswax, tribehenin, copernicia cerifera wax, glyceryl behenate, cetearyl alcohol, hydrogenated rapeseed oil, cera microcristallina, synthetic wax, and combinations thereof.

12. The solid anhydrous formulation for lip care of any of the preceding claims, wherein the butter mixture further comprises less than 50% of a combination of olus oil and hydrogenated vegetable oil and candelilla cera, and wherein all % are expressed in weight %.

13. The solid anhydrous formulation for lip care of any of the preceding claims, wherein the formulation comprises:
• from 45% to 55% of an oil mixture comprising at least at least 90% of Caprylic/Capric triglycerides;
• greater than 15% to less than 25% of a wax mixture comprising at least 20% of Glyceryl Behenate; and at least 40% of synthetic beeswax;
• from 25% to 35% of at least one butter, wherein the at least one butter comprises at least 50% of shea butter; and from 10% to less than 50% of a combination of olus oil and hydrogenated vegetable oil and candelilla cera,
wherein all % are expressed in weight %.

14. Use of a solid anhydrous formulation according to any of the preceding claims for the preparation of a lip stick balm.

15. Use of a solid anhydrous formulation according to any of the preceding claims for the make up or cosmetic care, for example hydration, of the lips.
